# EUROPEAN PATENT SPECIFICATION

(11) **EP 1 757 597 B1**
(45) Date of publication and mention of the grant of the patent: **11.04.2012**
(21) Application number: 05751076.0
(22) Date of filing: 14.06.2005
(51) Int. Cl.: C07D 307/93

(54) **METHOD FOR PRODUCING 2-OXO-1-PHENYL-3-OXABICYCLO[3.1.0]HEXANE**
HERSTELLUNGSVERFAHREN FÜR 2-OXO-1-PHENYL-3-OXABICYCLO[3.1.0]HEXAN
PROCÉDÉ DE PRODUCTION DU 2-OXO-1-PHÉNYL-3-OXABICYCLO[3.1.0]HEXANE

(30) Priority: 16.06.2004 JP 2004178957
(43) Date of publication of application: 28.02.2007
(73) Proprietor: Sumitomo Chemical Company, Limited, Tokyo 104-8260 (JP)
(72) Inventor: NIIMOTO, Yoshihide, Hyogo, 658-0014 (JP); KUMAZAWA, Hiroharu, 5691146 (JP); TOKUDA, Osamu, Nara, 631-0026 (JP); KAWAMI, Koh, Kurashiki-shi Okayama 7110903 (JP)
(74) Representative: Benson, John Everett
(86) International application number: PCT/JP2005/011221
(87) International publication number: WO 2005/123709

(56) References cited:
- EP-A- 1 767 522
- FR-A1- 2 302 994
- CASADIO S ET AL: "1 Phenylhydroxymethyl 2 cyclopropane carboxylic acid and derivatives./Acide Phenyl-1-Hydroxymethyl-2-Cclopropane Carboxylique et derives/" BOLLETTINO CHIMICO FARMACEUTICO, SOCIETA EDITORIALE FARMACEUTICA, MILANO, IT, vol. 117, no. 6, 1 January 1978 (1978-01-01), pages 331-342, XP008085513 ISSN: 0006-6648
- SHUTO S ET AL: "(PLUS OR MINUS)-(Z)-2-(AMINOMETHYL)-1-PHENYLCYCLOPR OPANECARBOXAMIDE DERIVATIVES AS A NEW PROTOTYPE OF NMDA RECEPTOR ANTAGONISTS" JOURNAL OF MEDICINAL CHEMISTRY, US AMERICAN CHEMICAL SOCIETY. WASHINGTON, vol. 38, no. 15, 21 July 1995 (1995-07-21), pages 2964-2968, XP000857416 ISSN: 0022-2623
- LANGER P ET AL: "Chemo-, regio-, and diastereoselective synthesis of functionalized cyclopropanes by cyclization of dilithiated nitriles with epibromohydrin" ORGANIC LETTERS, AMERICAN CHEMICAL SOCIETY, COLUMBUS, OH; US, vol. 3, no. 24, 29 November 2001 (2001-11-29), pages 3903-3905, XP002457493 ISSN: 1523-7060

## Description

### FIELD OF THE INVENTION

The present invention relates to a process for producing 2-oxo-1-phenyl-3-oxabicyclo[3.1.0]hexane.

### BACKGROUND OF THE INVENTION

2-Oxo-1-phenyl-3-oxabicyclo[3.1.0]hexane is useful as an intermediate for synthesizing (Z)-1-phenyl-1-diethylaminocarbonyl-2-aminomethylcyclopropane hydrochloride which is currently noticed as a therapeutic agent for depression.

The process for synthesizing 2-oxo-1-phenyl-3-oxabicyclo[3.1.0]hexane is exemplified by a process of reacting phenylacetonitrile with epichlorohydrin in the presence of sodium amide in benzene, hydrolyzing, and then acidifying (for example, FR2302994B, J. Org. Chem.,1996, 61, 915-923, Journal of Synthetic Organic Chemistry, Japan, vol. 55, No. 10, page 868-876 (1997), and the like). 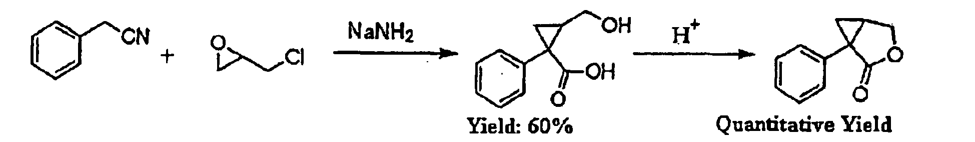 However, the above process uses sodium amide as a base. This causes problems that sodium amide is difficult to obtain industrially, hard for handling due to being easily hydrolyzed or aerially oxidized. This also causes problem that it generates explosive substances by the aerial oxidation.

Boll. Chim. Farm. 117 (1978) pages 331-342 describes synthesis of cis aryl-1-hydroxymethyl-2-cyclopropane carboxylic acids by action of carbon anions of arylacetonitriles on epichlorohydrin.

J. Med. Chem. 1995 38 pages 2964 to 2968 describes methods for preparing (±)-(*Z*)-2-(Aminomethyl)-1-phenylcyclopropane-*N,N*-diethylcarboxamide and its derivatives.

Organic Letters 2001 3(24) pages 3903 to 3905 describes the cyclization of 1,1-dianions with epibromohydrin, resulting in chemo-, regio- and diastereoselective formation of functionalized hydroxymethylcyclopropanes.

### SUMMARY OF THE INVENTION

The present invention intends to solve the above problems and to provide a safe, simple and industrially advantageous process for producing 2-oxo-1-phenyl-3-oxabicyclo[3.1.0]hexane.

After having diligently studied about the above problems, the present inventors have achieved the present invention.

Namely, the invention has the following aspects.
[1] A process for producing 2-oxo-1-phenyl-3-oxabicyclo[3.1.0]hexane which comprises reacting phenylacetonitrile with epichlorohydrin in the presence of sodium hydride in a solvent comprising an aprotic polar solvent which is at least one kind selected from the group consisting of N,N-dimethylformamide, N,N'-dimethylimidazolidinone, N,N-dimethylacetamide and N-methylpyrrolidone; followed by alkali hydrolysis and acid treatment.
[2] The process according to [1], wherein the solvent is a mixed solvent consisting of said aprotic polar solvent and toluene.
[3] The process according to [1] or [2], wherein said aprotic polar solvent is N,N'-dimethylimidazoline.
[4] The process according to [1], wherein the amount of epichlorohydrin is at least 1 gram equivalent per 1 gram equivalent of phenylacetonitrile
[5] The process according to [1], wherein the amount of sodium hydride is 1.1 to 3 gram equivalent per 1 gram equivalent of phenylacetonitrile.
[6] The process according to [1], wherein the reaction temperature is in a range of from -10 to +50°C.
[7] The process according to [1], wherein the reaction liquid obtained by the reaction of phenylacetonitrile with epichlorohydrin is subjected to alkali-hydrolysis and then the liquid obtained by the alkali-hydrolysis is subjected to acid-treatment.
[8] The process according to [7], wherein the liquid obtained by the alkali-hydrolysis is an aqueous layer separated from the reaction liquid obtained by alkali-hydrolysis.
[9] The process according to [8], wherein the acid treatment is carried out in the co-presence of a hydrophobic solvent.

### DESCRIPTION OF PREFERRED EMBODIMENTS

The present invention is explained in detail as follows.
The process for producing 2-oxo-1-phenyl-3-oxabicyclo[3.1.0]hexane of the present invention includes reacting phenylacetonitrile with epichlorohydrin in the presence of sodium hydride. After the completion of the reaction, the reaction product is further subjected to conventional alkali-hydrolysis and acid-treatment to obtain 2-oxo-1-phenyl-3-oxabicyclo[3.1.0]hexane.

### Reaction of phenylacetonitrile with epichlorohydrin

In the present reaction, sodium hydride is used as a base. The amount of sodium hydride used is preferably 1.1 to 3 gram equivalent per 1 gram equivalent of phenylacetonitrile, and more preferably 1.2 to 2 gram equivalent. Sodium hydride is commercially available in a suspended form in a mineral oil, which is more stable to hydrolysis or aerial oxidation and easier in handling compared to sodium amide used in the conventional production processes, and sodium hydride never generates explosive substance.

In the present reaction, solvent is used, and the solvent comprises an aprotic polar solvent which is at least one kind selected from N,N-dimethylformamide (hereinafter, occasionally referred to as DMF), N,N'-dimethylimidazolidinone(hereinafter, occasionally referred to as DMI), N,N-dimethylacetamide (hereinafter, occasionally referred to as DMAC), and N-methylpyrrolidone (hereinafter, occasionally referred to as NMP). These may be used alone or as a mixture of two or more kinds thereof. When being used as a mixture, a mixing ratio thereof is not particularly limited. Alternatively, the solvent may include a mixed solvent consisting of said aprotic polar solvent and toluene. In this case, the content of the aprotic polar solvent in the mixed solvent is preferably 50% by weight or more, and more preferably 75% by weight or more, in view of preventing decrease of a rate of anionization of phenylacetonitrile by the sodium hydride:

The amount of the solvent is generally 1 to 10 kg per 1 kg of phenylacetonitrile, and preferably 2 to 4 kg.

In the present reaction, in view of preventing decrease of yield, the amount of epichlorohydrin is usually at least 1 gram equivalent per 1 gram equivalent of phenylacetonitrile. On the other hand, no problem occurs when un-reacted epichlorohydrin (an excess amount to phenylacetonitrile) may remain in the reaction system. However, in view of economy, the amount of epichlorohydrin is 1 to 2 gram equivalent per 1 gram equivalent of phenylacetonitrile.

In view of controlling the reaction rate and preventing by-production of impurities, the reaction temperature is preferably in the range of -10 to +50°C, and more preferably in the range of 10 to 20°C. The reaction time is usually 0.5 to 20 hours, though it may change depending on the reaction temperature.

In the reaction of phenylacetonitrile with epichlorohydrin in the presence of the sodium hydride mentioned above, the reaction may be carried out by mixing sodium hydride, phenylacetonitrile, and epichlorohydrin, the mixing order thereof is not particularly limited.

For example, sodium hydride, phenylacetonitrile, and epichlorohydrin may be added to the solvent (the order of addition is not particularly limited), or may be suspended or dissolved with the solvent respectively, and then the respective suspensions or solutions can be mixed (the order of mixing is not particularly limited).

The progress of the reaction can be confirmed by checking the change of phenylacetonitrile concentration in the reaction system, and the completion of the reaction can be confirmed by disappearance of phenylacetonitrile.

After the completion of the reaction mentioned above, 2-oxo-1-phenyl-3-oxabicyclo[3.1.0]hexane is obtained by alkali hydrolysis and acid-treatment.

### The Alkali-Hydrolysis

Alkalis used in the present alkali hydrolysis are not particularly limited. Examples thereof include potassium hydroxide, sodium hydroxide, lithium hydroxide, calcium hydroxide, and the like. Among them, potassium hydroxide and sodium hydroxide are preferable in view of their cost. The amount of the alkali used is 1 to 3 gram equivalent per 1 gram equivalent of phenylacetonitrile, and preferably 1.5 to 2 gram equivalent. A reaction temperature is generally at 30 to 100°C, and preferably at a refluxing temperature. A reaction time is usually 5 to 30 hours.

Furthermore, the alkali-hydrolysis may be carried out with addition of a phase-transfer catalyst. Examples of the phase-transfer catalyst include tetrabutylammonium sulfate, tetrabutylammonium chloride, tetrabutylammonium bromide, benzyltrimethylammonium sulfate, benzyltrimethylammonium chloride, benzyltrimethylammonium bromide, and the like. The amount of the phase-transfer catalyst is 0.005 to 0.05 gram equivalent per 1 gram equivalent of phenylacetonitrile.

### The Add-Treatment

The acids used for the acid-treatment are not particularly limited. Examples thereof include hydrochloric acid, sulfuric acid, phosphoric acid, and the like. The acid-treatment is carried out by adding the acid to the liquid obtained by the above-mentioned alkali-hydrolysis, generally with adjusting pH of the reaction system to 0 to 4, and preferably 0 to 2. As the liquid obtained by the alkali-hydrolysis and to be subjected to the acid-treatment, the reaction mixture may be used as itself; preferably, aqueous layer may be used which is obtained after eliminating oil layer from the reaction mixture by a phase separation or the like. More preferably the aqueous layer is more preferably subjected to the acid-treatment in the co-presence of the hydrophobic solvent by adding hydrophobic solvent (for example, hydrocarbon-based solvents such as toluene, and the like, ketone-based solvents such as methylisobutylketone, and the like, ether-based solvents such as methyl tert-butyl ether, and the like) with the aqueous layer. A temperature in the acid-treatment is generally 10 to 100°C, and preferably 60 to 70°C. A treatment time is usually 0.5 to 5 hours. The amount of hydrophobic solvent when used is usually 1 to 10 kg per 1 kg of phenylacetonitrile.

The intended compound (2-oxo-1-phenyl-3-oxabicyclo[3.1.0]hexane) produced by the above mentioned process can be isolated from the acid-treated liquid by usual after-treatment (liquid separation, washing, solvent distillation, and the like) usually in a form of oily substance, and, depending on requirement, may be isolated as crystals by crystallization. Furthermore, the isolated crystals may be purified, depending on requirement, by conventional means such as recrystallization, chromatography, and the like.

2-Oxo-1-phenyl-3-oxabicyclo[3.1.0]hexane produced by the process of the present invention can be lead to (Z)-1-phenyl-1-diethylaminocarbonyl-2-aminomethyl-cyclopropane hydrochloride as a therapeutic agent for depression according to a method, for example, described in JPH05-67136-B.

The invention will be explained in more detail according to Example, but should not be construed to be limited thereto.

### Example 1

### Production of 2-xo-1-phenyl-3-oxabicyclo[3.1.0]hexane

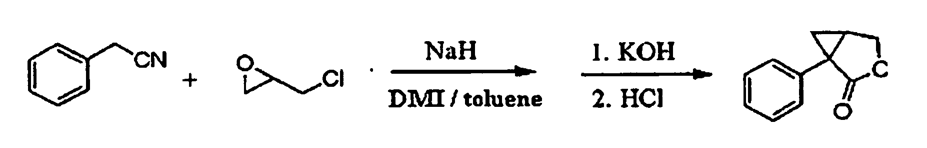

To a mixed solvent of toluene (26.0 kg) and N,N'-dimethylimidazolidinone (94.9 kg), 60% sodium hydride (27.2 kg; 683 mol) was added, and phenylacetonitrile (40.2 kg; 343 mol) was subsequently dropped therein at 10 to 20 °C. And then, a mixture of epichlorohydrin (31.7 kg; 343 mol) and toluene (26.0 kg) was dropped therein at 10 to 20°C and then stirred. After confirming the disappearance of the raw materials, methanol (22.0 kg) and water (120.6 kg) were added therein to be subjected to phase separation and washing.

The organic layer obtained was added with 24% aqueous solution of potassium hydroxide (159.1 kg) and tetrabutylammonium sulfate (1.1 kg), and the added mixture was subjected to reaction under refluxing. After the completion of the reaction, the organic layer was removed by a phase separation, and then the aqueous layer was added with toluene (69.6 kg) and 35% hydrochloric acid (78.7 kg), followed by stirring at 60 to 70°C for 2 hours. After a phase separation, organic layer was further washed twice with 8% aqueous solution of sodium hydrogen carbonate and twice with water. The organic layer obtained was concentrated under a reduced pressure to obtain 40.7 kg (yield 68.1%) of 2-oxo-1-phenyl-3-oxabicyclo[3.1.0]hexane in the form of light-yellow oily substance.

Without further purification, the oily substance obtained can be used for the production of (Z)-1-phenyl-1-diethylaminocarbonyl-2-aminomethylcyclopropane hydrochloride.

A portion of 2-oxo-1-phenyl-3-oxabicyclo[3.1.0]hexane obtained was taken out to measure its physical properties.
¹H-NMR (CDCl₃,400MHz) d 1.35 (1H, t, J=4.8Hz), 1.63 (1H, dd, J=4.8,8.0Hz), 2.54 (1H, ddd, J=4.8, 4.8, 8.0Hz), 4.28 (1H, d, J=9.2Hz), 4.45 (1H, dd, J=4.8, 9.2Hz), 7.22-7.42 (5H, m)

According to the method of the present invention, 2-oxo-1-phenyl-3-oxabicyclo[3.1.0]hexane can be produced safely, easily and industrially advantageously by using sodium hydride as a base in the reaction of phenylacetonitrile and epichlorohydrin.

## Claims

1. A process for producing 2-oxo-1-phenyl-3-oxabicyclo[3.1.0]hexane which comprises reacting phenylacetonitrile with epichlorohydrin in the presence of sodium hydride in a solvent comprising an aprotic polar solvent which is at least one kind selected from the group consisting of N,N-dimethylformamide, N,N'-dimethylimidazolidinone, N,N'-dimethylacetamide and N-methylpyrrolidone; followed by alkali hydrolysis and acid treatment.

2. The process according to claim 1, wherein the solvent is a mixed solvent consisting of said aprotic polar solvent and toluene.

3. The process according to claim 1 or 2, wherein said aprotic polar solvent is N,N'-dimethylimidazoline.

4. The process according to claim 1, wherein the amount of epichlorohydrin is at least 1 gram equivalent per 1 gram equivalent of phenylacetonitrile.

5. The process according to claim 1, wherein the amount of sodium hydride is 1.1 to 3 gram equivalent per 1 gram equivalent of phenylacetonitrile.

6. The process according to claim 1, wherein the reaction temperature is in a range of from -10 to +50°C.

7. The process according to claim 1, wherein the reaction liquid obtained by the reaction of phenylacetonitrile with epichlorohydrin is subjected to alkali-hydrolysis and then the liquid obtained by the alkali-hydrolysis is subjected to acid-treatment.

8. The process according to claim 7, wherein the liquid obtained by the alkali-hydrolysis is an aqueous layer separated from the reaction liquid obtained by alkali-hydrolysis.

9. The process according to claim 8, wherein the acid treatment is carried out in the co-presence of a hydrophobic solvent.

## Patentansprüche

1. Verfahren zur Herstellung von 2-Oxo-1-phenyl-3-oxabicyclo[3.1.0]hexan, welches umfasst: Umsetzen von Phenylacetonitril mit Epichlorhydrin in der Gegenwart von Natriumhydrid in einem Lösungsmittel, das ein aprotisches polares Lösungsmittel umfasst, welches wenigstens eine Art ist, die aus der Gruppe bestehend aus N,N-Dimethylformamid, N,N'-Dimethylimidazolidinon, N,N'-Dimethylacetamid und N-Methylpyrrolidon ausgewählt ist; gefolgt von Alkali-Hydrolyse und Säurebehandlung.

2. Verfahren nach Anspruch 1, wobei das Lösungsmittel ein gemischtes Lösungsmittel ist, das aus dem aprotischen polaren Lösungsmittel und Toluol besteht.

3. Verfahren nach Anspruch 1 oder 2, wobei es sich bei dem aprotischen polaren Lösungsmittel um N,N'-Dimethylimidazolin handelt.

4. Verfahren nach Anspruch 1, wobei die Menge Epichlorhydrin mindestens 1 Grammäquivalent pro 1 Grammäquivalent Phenylacetonitril beträgt.

5. Verfahren nach Anspruch 1, wobei die Menge Natriumhydrid 1,1 bis 3 Grammäquivalente pro 1 Grammäquivalent Phenylacetonitril beträgt.

6. Verfahren nach Anspruch 1, wobei die Reaktionstemperatur in einem Bereich von -10 bis +50 °C liegt.

7. Verfahren nach Anspruch 1, wobei die Reaktionsflüssigkeit, die durch die Umsetzung von Phenylacetonitril mit Epichlorhydrin erhalten wird, einer Alkali-Hydrolyse unterzogen wird, und die Flüssigkeit, die durch die Alkali-Hydrolyse erhalten wird, anschließend einer Säurebehandlung unterzogen wird.

8. Verfahren nach Anspruch 7, wobei die durch die Alkali-Hydrolyse erhaltene Flüssigkeit eine wässrige Schicht ist, die aus der durch Alkali-Hydrolyse erhaltenen Reaktionsflüssigkeit abgeschieden wird.

9. Verfahren nach Anspruch 8, wobei die Säurebehandlung in der gleichzeitigen Gegenwart eines hydrophoben Lösungsmittels durchgeführt wird.

## Revendications

1. Procédé de production de 2-oxo-1-phényl-3-oxabicyclo[3.1.0]hexane, qui comprend : la réaction de phénylacétonitrile avec de l'épichlorhydrine en présence d'hydrure de sodium dans un solvant comprenant un solvant polaire aprotique qui est au moins un type choisi dans le groupe constitué par le N,N-diméthylformamide, la N,N'-diméthylimidazolidinone, le N,N'-diméthylacétamide et la N-méthylpyrrolidone ; suivie d'une hydrolyse alcaline et d'un traitement acide.

2. Procédé selon la revendication 1, dans lequel le solvant est un solvant composé constitué dudit solvant polaire aprotique et de toluène.

3. Procédé selon la revendication 1 ou 2, dans lequel ledit solvant polaire aprotique est la N,N'-diméthylimidazoline.

4. Procédé selon la revendication 1, dans lequel la quantité d'épichlorhydrine est d'au moins 1 équivalent gramme pour 1 équivalent gramme de phénylacétonitrile.

5. Procédé selon la revendication 1, dans lequel la quantité d'hydrure de sodium est de 1,1 à 3 équivalents grammes pour 1 équivalent gramme de phénylacétonitrile.

6. Procédé selon la revendication 1, dans lequel la température de réaction est dans une plage allant de -10 à +50°C.

7. Procédé selon la revendication 1, dans lequel le liquide de réaction obtenu par la réaction de phénylacétonitrile avec l'épichlorhydrine est soumis à une hydrolyse alcaline et ensuite le liquide obtenu par l'hydrolyse alcaline est soumis à un traitement par un acide.

8. Procédé selon la revendication 7, dans lequel le liquide obtenu par l'hydrolyse alcaline est une couche aqueuse séparée du liquide de réaction obtenu par hydrolyse alcaline.

9. Procédé selon la revendication 8, dans lequel le traitement acide est réalisé en présence conjointe d'un solvant hydrophobe.
